# EUROPÄISCHE PATENTANMELDUNG

(11) **EP 3 363 417 A1**
(43) Veröffentlichungstag der Anmeldung: **22.08.2018**
(21) Anmeldenummer: 17156388.5
(22) Anmeldetag: 16.02.2017
(51) Int. Cl.: A61F 9/06, A42B 3/04, B23K 9/095, B23K 37/00

(54) **BLENDSCHUTZVORRICHTUNG, INSBESONDERE SCHWEISSHELM**

(71) Anmelder: optrel Holding AG, 9050 Appenzeil (CH)
(72) Erfinder: HOFER KRANER, Ramon, 9100 Herisau (CH); PITTORINO, Tindaro, 9470 Buchs SG (CH); SCHREIBER, Olaf, 9470 Buchs SG (CH)
(74) Vertreter: Daub, Thomas

(57) **Zusammenfassung**

Die Erfindung geht aus von einer Blendschutzvorrichtung, insbesondere von einem Schweißhelm, mit zumindest einem optischen Blendschutzfilter (12a; 12b), mit zumindest einer Blendschutzsensoreinheit (14a; 14b), welche zu einer Erfassung eines Arbeitszustands und einer davon abhängigen Ansteuerung einer Durchlässigkeit des Blendschutzfilters (12a; 12b) vorgesehen ist, und mit zumindest einer Kommunikationseinheit (16a; 16b) zu einer Kommunikation mit einem von der einen Blendschutzvorrichtung (10a; 10b) getrennten Bedienerendgerät (18a; 18b; 18c; 18d; 18e; 18f).

Es wird vorgeschlagen, dass die zumindest eine Blendschutzsensoreinheit (14a; 14b) zumindest einen Sensor (20a) aufweist, der zumindest teilweise einstückig mit einem Sensor (22a) der Kommunikationseinheit (16a; 16b) ausgebildet ist.

## Beschreibung

### Stand der Technik

Die Erfindung betrifft eine Blendschutzvorrichtung.

Es ist bereits eine Blendschutzvorrichtung, insbesondere ein Schweißhelm, mit zumindest einem optischen Blendschutzfilter, mit zumindest einer Blendschutzsensoreinheit, welche zu einer Erfassung eines Arbeitszustands und einer davon abhängigen Ansteuerung einer Durchlässigkeit des Blendschutzfilters vorgesehen ist, und mit zumindest einer Kommunikationseinheit zu einer Kommunikation mit einem von der einen Blendschutzvorrichtung getrennten Bedienerendgerät, vorgeschlagen worden.

Blendschutzvorrichtungen, insbesondere Schweißhelme, haben als primäres Ziel, insbesondere das menschliche Auge vor Schäden durch Blendung, die beispielsweise während des Schweissprozesses entstehen, zu schützen. Ihr Vorteil gegenüber konventionellen Schutzgläsern besteht insbesondere in der automatischen Verdunkelung wenige Millisekunden nach dem Zünden des Lichtbogens. Eine Blendschutzvorrichtung muss daher zum Positionieren der Elektrode bzw. des Schweissbrenners nicht hochgeklappt oder abgenommen werden. Die meisten heute auf dem Markt verfügbaren Blendschutzvorrichtungen detektieren das Zünden des Schweissbogens mittels eines Fotosensors und - abhängig von der empfangenen Lichtstärke - steuern sie die Abdunkelung eines LCD Elements, wobei nicht sichtbares Licht mittels Infrarot- und Ultraviolettfilter abgeschirmt wird. Um die Blendschutzvorrichtung an alle möglichen Schweissverfahren sowie Schweisslichtintensitäten anpassen zu können, besteht insbesondere teilweise die Möglichkeit, Parameter in der Blendschutzvorrichtung einzustellen. Des Weiteren ist es von Vorteil, für die Schweisshelmhersteller die Möglichkeit zu haben, Software-Updates durchführen zu können. Die Einstellung der Parameter bzw. die Durchführung der Software-Updates werden heute abhängig von der Preisklasse der Blendschutzvorrichtung über manuelle Einstellungen, eine RF Kommunikation oder eine Kabelverbindung erreicht. Bei der manuellen Einstellung werden die für den normalen Betrieb notwendigen Einstellungen manuell an der Blendschutzvorrichtung vorgenommen. Es ist kein Software-Update möglich. Bei der RF Kommunikation wird zur Parametrierung der Blendschutzvorrichtung eine RF-Kommunikation, wie beispielsweise Bluetooth, in der Blendschutzvorrichtung verbaut. Damit besteht die Möglichkeit, mit der Blendschutzvorrichtung zu kommunizieren. Es können recht komfortabel die Benutzereinstellungen verändert werden. Es ist auch ein Softwaredownload der Firmware möglich. Andererseits fallen bei dieser Variante zusätzliche Kosten im Entwicklungsprozess an. Für das Inverkehrbringen eines neuen Produktes sind RF-Zulassungsmessungen in einem akkreditierten Messlabor zwingend erforderlich. Daneben ist in der Blendschutzvorrichtung ein HF-Modul einzusetzen, wodurch weitere Kosten entstehen. Das HF-Modul braucht allgemein Strom und daher muss in der Blendschutzvorrichtung eine große Batterie oder entsprechende intelligente Stromsparfunktionen verbaut werden. Bei einer Kabelverbindung ist zur Kommunikation ein Steckverbinder an der Blendschutzvorrichtung vorgesehen. Somit kann die Blendschutzvorrichtung parametriert werden und es ist auch ein Software-Update mit einem PC/Notebook möglich. Es ist bei dieser Variante ein entsprechendes Kabel notwendig.

Die Aufgabe der Erfindung besteht insbesondere darin, eine gattungsgemäße Vorrichtung mit verbesserten Eigenschaften hinsichtlich einer Komplexität sowie einer Kompaktheit bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Blendschutzvorrichtung, insbesondere von einem Schweißhelm, mit zumindest einem optischen Blendschutzfilter, mit zumindest einer Blendschutzsensoreinheit, welche zu einer Erfassung eines Arbeitszustands und einer davon abhängigen Ansteuerung einer Durchlässigkeit des Blendschutzfilters vorgesehen ist, und mit zumindest einer Kommunikationseinheit zu einer Kommunikation mit einem von der einen Blendschutzvorrichtung getrennten Bedienerendgerät.

Es wird vorgeschlagen, dass die zumindest eine Blendschutzsensoreinheit zumindest einen Sensor aufweist, der zumindest teilweise einstückig mit einem Sensor der Kommunikationseinheit ausgebildet ist. Vorzugsweise wird der zumindest eine Sensor der Blendschutzsensoreinheit sowohl zumindest teilweise zu einer Erfassung eines Arbeitszustands als auch zumindest teilweise zu einer Kommunikation genutzt. Unter einer "Blendschutzvorrichtung" soll in diesem Zusammenhang insbesondere eine Vorrichtung verstanden werden, welche zu einem Schutz eines Benutzers vor zu großer Helligkeit und/oder vor Funken vorgesehen ist. Vorzugsweise soll darunter insbesondere eine Vorrichtung verstanden werden, welche zu einem Schutz von Augen und/oder einem Gesichtsbereich eines Benutzers während eines Schweiß- und/oder Schleifprozesses dient. Bevorzugt soll darunter insbesondere eine Blendschutzvorrichtung verstanden werden, welche insbesondere zu einem Schutz der Augen eines Benutzers zumindest während eines Schweißprozesses dient. Es sind verschiedene, einem Fachmann als sinnvoll erscheinende Ausgestaltungen einer Blendschutzvorrichtungen denkbar, wie beispielsweise als Schweißhelm, -schirm, -maske und/oder -schild. Ferner soll in diesem Zusammenhang unter einem "optischen Blendschutzfilter" insbesondere ein optischer Filter verstanden werden, welcher insbesondere ein Schutzglas und/oder ein Kunststoffschutzglas bildet. Vorzugsweise soll darunter insbesondere ein optischer Filter verstanden werden, dessen Lichtdurchlässigkeit einstellbar ausgeführt ist. Bevorzugt soll darunter insbesondere ein optischer Schweißschutzfilter mit einer automatischen Verdunkelung verstanden werden. Besonders bevorzugt weist der Blendschutzfilter zumindest eine in der Transmission schaltbare Flüssigkristallebene auf. Es sind verschiedene, einem Fachmann als sinnvoll erscheinende Ausgestaltungen des optischen Blendschutzfilters denkbar, insbesondere soll darunter jedoch ein ADF, auch "automatic darkening filter" oder "automatischer Schweißerschutzfilter" genannt, verstanden werden. Unter einer "Blendschutzsensoreinheit" soll in diesem Zusammenhang insbesondere eine Einheit mit zumindest einem Sensor verstanden werden, welche in zumindest einem Betriebszustand zu einer Erfassung eines Arbeitszustands, insbesondere eines von einem Träger genutzten Geräts, bevorzugt eines Schweißgeräts, vorgesehen ist. Vorzugsweise soll darunter insbesondere eine Einheit verstanden werden, welche dazu vorgesehen ist, einen auftretenden Lichtbogen eines von dem Träger genutzten Geräts, insbesondere eines Schweißgeräts, zu erfassen. Dabei soll unter einem "Sensor" insbesondere ein Element verstanden werden, das dazu vorgesehen ist, zumindest eine Kenngröße und/oder eine physikalische Eigenschaft aufzunehmen, wobei die Aufnahme aktiv, wie insbesondere durch Erzeugen und Aussenden eines elektrischen Messsignals, und/oder passiv, wie insbesondere durch eine Erfassung von Eigenschaftsänderungen eines Sensorbauteils, stattfinden kann. Es sind verschiedene, dem Fachmann als sinnvoll erscheinende Sensoren denkbar. Vorzugsweise weist die Sensoreinheit insbesondere zumindest eine Photodiode oder Solarzelle auf. Bevorzugt ist die Photodiode oder Solarzelle in zumindest einem Betriebszustand zumindest zu einer optischen Detektion eines Lichtbogens, insbesondere während eines Schweißvorgangs, vorgesehen. Ferner soll in diesem Zusammenhang unter einer "Kommunikationseinheit" insbesondere eine Einheit verstanden werden, welche zu einer Bereitstellung einer insbesondere kabellosen Kommunikation mit einem externen Gerät, insbesondere mit einem Bedienerendgerät, vorgesehen ist. Vorzugs-weise weist die Kommunikationseinheit zu einer Kommunikation mit dem externen Gerät, insbesondere mit dem Bedienerendgerät, zumindest eine Schnittstelle auf. Vorzugsweise soll unter einer Kommunikationseinheit insbesondere eine Einheit verstanden werden, welche zu einem Austausch von Daten vorgesehen ist. Insbesondere weist die Kommunikationseinheit zumindest einen Informationseingang und zumindest einen Informationsausgang auf. Vorzugsweise weist die Kommunikationseinheit zumindest zwei Informationseingänge und zumindest zwei Informationsausgänge auf, wobei jeweils zumindest ein Informationseingang und zumindest ein Informationsausgang zu einer Verbindung mit einem physischen System, insbesondere dem externen Gerät, vorgesehen sind. Besonders bevorzugt soll darunter eine Schnittstelle zwischen zumindest zwei physischen Systemen, wie insbesondere zwischen dem Bedienerendgerät und der Blendschutzvorrichtung, verstanden werden. Es sind verschiedene, dem Fachmann als sinnvoll erscheinende Kommunikationseinheiten denkbar, insbesondere soll darunter jedoch eine drahtlose Schnittstelle verstanden werden. Unter einer "Kommunikation" soll in diesem Zusammenhang insbesondere ein vorzugsweise bidirektionaler, drahtloser Austausch von Daten mittels einer Übertragung von Signalen verstanden werden. Unter einem "Bedienerendgerät" soll in diesem Zusammenhang insbesondere ein Gerät zu einer direkten oder indirekten Kommunikation mit einem Bediener verstanden werden. Vorzugsweise soll darunter insbesondere ein mobiles Gerät zu Kommunikation mit einem Bediener verstanden werden. Es sind verschiedene, einem Fachmann als sinnvoll erscheinende Bedienerendgeräte denkbar, insbesondere soll darunter jedoch ein Computer, ein Smartphone, ein Tablet-PC und/oder ein Wearable Computer verstanden werden. Als Bedienerendgerät beispielsweise zum Parametrieren der Blendschutzvorrichtung wird insbesondere ein Smartphone oder Tablet-Computer favorisiert. Am Markt existieren eine Reihe von Smartphone- und Tablet-Systemen. Neben anderen sind die folgenden Sensoren und Aktoren in praktisch jedem Smartphone vorhanden: Bewegungssensoren zur Erfassung von linearer Beschleunigung, Drehbeschleunigung, Schwerkraft und/oder Magnetfeld, Umgebungssensoren zur Erfassung von Temperatur, Druck, Luftfeuchtigkeit, Abstand und/oder Umgebungslicht, ein Blitzlicht und/oder Vibrationselemente. Daneben lässt sich zur Interaktion mit der Umgebung auch die Kamera und das Display verwenden. Die genannten Sensoren und/oder Aktoren lassen sich durch eine Applikation ansteuern und auslesen.

Darunter, dass der zumindest eine Sensor "teilweise einstückig" mit einem Sensor der Kommunikationseinheit ausgebildet ist, soll insbesondere verstanden werden, dass die Sensoren zumindest ein, insbesondere zumindest zwei, vorteilhaft zumindest drei gemeinsame Elemente aufweisen, die Bestandteile, insbesondere funktionell wichtiger Bestandteile, beider Sensoren sind. Vorzugsweise sind die Sensoren als ein Sensor ausgebildet. Unter "vorgesehen" soll insbesondere speziell programmiert, ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch die erfindungsgemäße Ausgestaltung der Blendschutzvorrichtung kann insbesondere eine Komplexität eines Aufbaues der Blendschutzvorrichtung gering gehalten werden. Vorzugsweise können dadurch vorteilhaft bestehende und benötigte Sensoren der Blendschutzvorrichtung neben einer Erfassung eines Arbeitszustands zumindest teilweise für eine Kommunikation genutzt werden. Hierdurch kann eine Anzahl von Bauteilen gering gehalten werden. Es kann eine vorteilhaft kostengünstige Blendschutzvorrichtung bereitgestellt werden. Ferner kann hierdurch eine vorteilhaft kompakte Blendschutzvorrichtung bereitgestellt werden. In bestehenden Blendschutzvorrichtungen, insbesondere Schweißhelmen, sind ein Lichtsensor zur Detektion des Schweißlichtes sowie ein LCD-Shutter zum Blendschutz des Bedieners standardmäßig vorhanden. Es gibt in der Blendschutzvorrichtung in der Regel keine weiteren verfügbaren Sensoren bzw. Aktoren.

Ferner wird vorgeschlagen, dass die zumindest eine Kommunikationseinheit in zumindest einem Betriebszustand dazu vorgesehen ist, eine Sensorkenngröße des zumindest einen Sensors der zumindest einen Blendschutzsensoreinheit zu einer Kommunikation zu nutzen. Vorzugsweise ist die zumindest eine Kommunikationseinheit in zumindest einem Betriebszustand dazu vorgesehen, eine durch den zumindest einen Sensor der zumindest einen Blendschutzsensoreinheit erfasste Sensorkenngröße zu einer Kommunikation zu nutzen. Bevorzugt wird die Sensorkenngröße des zumindest einen Sensors der zumindest einen Blendschutzsensoreinheit sowohl zu einer Erfassung eines Arbeitszustands als auch zu einer Kommunikation genutzt. Dadurch kann der zumindest eine Sensor der zumindest einen Blendschutzsensoreinheit vorteilhaft für verschiedene Funktionen genutzt werden. Es kann insbesondere auf weitere Sensoren verzichtet werden. Insbesondere können dadurch auf zusätzliche Zertifizierungskosten, wie beispielsweise bei einem Einsatz eines separaten, zusätzlichen Funkmoduls, entfallen. Ferner kann dadurch zudem eine Komplexität einer Recheneinheit der Blendschutzvorrichtung gering gehalten werden.

Des Weiteren wird vorgeschlagen, dass der zumindest eine Sensor der zumindest einen Blendschutzsensoreinheit bifunktional ausgebildet ist und zumindest eine Arbeitszustandserfassungsfunktion und zumindest Kommunikationsfunktion aufweist. Unter einer "Arbeitszustandserfassungsfunktion" soll in diesem Zusammenhang insbesondere eine Funktion des Sensors verstanden werden, bei welcher der Sensor zu einer direkten Erfassung einer Kenngröße vorgesehen ist, welche auf einen Arbeitszustand rückschließen lässt. Vorzugsweise ist der Sensor in zumindest einem Betriebszustand zu einer direkten Erfassung eines auftretenden Lichtbogens eines von dem Träger genutzten Geräts, insbesondere eines Schweißgeräts, vorgesehen. Unter einer "Kommunikationsfunktion" soll in diesem Zusammenhang insbesondere eine Funktion des Sensors verstanden werden, bei welcher der Sensor insbesondere zu einer direkten Erfassung eines Kommunikationssignals vorgesehen ist. Vorzugsweise ist der Sensor in zumindest einem Betriebszustand zu einer direkten Erfassung eines Signals vorgesehen, welches zu einer Übertragung von Daten, insbesondere auf die Blendschutzvorrichtung, genutzt wird. Dadurch kann der zumindest eine Sensor der zumindest einen Blendschutzsensoreinheit vorteilhaft für verschiedene Funktionen genutzt werden. Es kann insbesondere auf weitere Sensoren verzichtet werden.

Es wird ferner vorgeschlagen, dass der zumindest eine Sensor der zumindest einen Blendschutzsensoreinheit in zumindest einem Empfangszustand dazu vorgesehen ist, mittels Licht übertragene Daten des Bedienerendgeräts zu empfangen. Vorzugsweise ist der zumindest eine Sensor der zumindest einen Blendschutzsensoreinheit in zumindest einem Empfangszustand dazu vorgesehen, kodierte Daten, welche von dem Bedienerendgerät mittels Licht, insbesondere mittels Lichtimpulsen, ausgesendet werden, zu empfangen. Bevorzugt ist der zumindest eine Sensor dazu vorgesehen, das von dem Bedienerendgerät ausgesendete Licht zu erfassen und weiterzuleiten. Unter "mittels Licht übertragenen Daten" sollen in diesem Zusammenhang insbesondere digitale Daten, welche vorzugsweise eine konkrete Information enthalten, verstanden werden, welche zu einer Übertragung in Licht, vorzugsweise in Lichtimpulse, umgewandelt werden, welches von einem Empfangssystem interpretiert werden kann, wobei die digitalen Daten von dem Empfangssystem ausgelesen werden können. Vorzugsweise können die digitalen Daten beispielsweise in Form von Lichtimpulsen übertragen werden, wobei mittels der Lichtimpulse beispielsweise eine Folge von Einsen und Nullen dargestellt werden kann. Hierdurch kann insbesondere eine vorteilhafte Datenübertragung bereitgestellt werden, bei welcher Daten mittels des zumindest einen Sensors der zumindest einen Blendschutzsensoreinheit erfasst werden können. Es kann insbesondere mit geringen Hardwarekosten eine Kommunikation bereitgestellt werden. Ferner kann daher eine Implementierung in eine bestehende Blendschutzvorrichtung unter Nutzung der vorhandenen Peripherie ermöglicht werden. Zudem kann dadurch eine Parametrierung der Blendschutzvorrichtung im Betrieb ermöglicht werden. Unter der Voraussetzung, dass eine ausreichende Übertragungsgeschwindigkeit erreicht wird, können dadurch auch Updates auf die Blendschutzvorrichtung aufgespielt werden.

Es wird weiter vorgeschlagen, dass der zumindest eine Sensor der zumindest einen Blendschutzsensoreinheit dazu vorgesehen ist, mittels des Bedienerendgeräts erzeugte Lichtimpulse zu erfassen und zu einer Auswertung an die zumindest eine Kommunikationseinheit zu übertragen. Vorzugsweise werden die Lichtimpulse von dem zumindest einen Sensor der Blendschutzsensoreinheit erfasst und ein Messsignal wird an die Kommunikationseinheit übertragen. Bevorzugt ist die Kommunikationseinheit in zumindest einem Betriebszustand dazu vorgesehen, die Messsignale des zumindest einen Sensors auszuwerten und eine hinterlegte Information auszulesen. Besonders bevorzugt ist die Kommunikationseinheit zu einer Dekodierung der Lichtimpulse des Bedienerendgeräts vorgesehen. Dadurch kann insbesondere eine vorteilhaft zuverlässige und schnelle Datenübertragung ermöglicht werden. Dadurch kann insbesondere eine Datenübertragung ermöglicht werden, welche sinnvoll von dem zumindest einen Sensor der Blendschutzsensoreinheit erfasst werden kann, insbesondere auch, da der Sensor für eine Arbeitszustandserfassungsfunktion bereits eine schnelle Reaktionszeit benötigt.

Ferner wird vorgeschlagen, dass der zumindest eine Sensor der zumindest einen Blendschutzsensoreinheit von einer Photodiode oder einer Solarzelle gebildet ist. Dadurch kann ein vorteilhaft zuverlässiger Sensor mit einer vorteilhaft hohen Energieausbeute bereitgestellt werden. Ferner kann insbesondere auf eine externe Energieversorgung, wie beispielsweise mittels einer Batterie und/oder mittels eines Akkumulators, verzichtet werden. Bei der Verwendung einer Photodiode kann insbesondere eine Sensoreinheit bereitgestellt werden, mittels welcher insbesondere auch Licht in einem nicht sichtbaren Bereich, wie insbesondere Infrarotlicht, erfasst und vorzugsweise verwertet werden kann.

Des Weiteren wird vorgeschlagen, dass die zumindest eine Kommunikationseinheit in zumindest einem Übertragungszustand zu einer Übertragung von Daten auf das Bedienerendgerät zu einer Ansteuerung einer Durchlässigkeit des Blendschutzfilters vorgesehen ist. Vorzugsweise ist die Kommunikationseinheit in dem zumindest einen Übertragungszustand dazu vorgesehen, zu übertragende Daten kodiert durch eine Veränderung einer Durchlässigkeit des Blendschutzfilters darzustellen. Darunter, dass die Kommunikationseinheit Daten "durch eine Veränderung einer Durchlässigkeit des Blendschutzfilters" darstellt, soll in diesem Zusammenhang insbesondere verstanden werden, dass digitale Daten, welche vorzugsweise eine konkrete Information enthalten, zu einer Übertragung in Abdunklungsimpulse umgewandelt werden, welche von einem Empfangssystem interpretiert werden können, wobei die digitalen Daten von dem Empfangssystem ausgelesen werden können. Vorzugsweise können die digitalen Daten beispielsweise in Form von Abdunklungsimpulsen übertragen werden, wobei mittels der Abdunklungsimpulse beispielsweise eine Folge von Einsen und Nullen dargestellt werden kann. Dadurch kann insbesondere eine Datenübertragung mittels des für einen regulären Betrieb benötigten Blendschutzfilters ermöglicht werden. Hierdurch kann insbesondere eine Anzahl von Bauteilen gering gehalten werden. Es kann insbesondere ein vorteilhaft kompakter Aufbau der Blendschutzvorrichtung erreicht werden.

Ferner geht die Erfindung aus von einem System mit der zumindest einen Blendschutzvorrichtung und mit zumindest einem von der zumindest einen Blendschutzvorrichtung getrennten Bedienerendgerät, welches zumindest eine Ein- und/oder Ausgabeeinheit zu einer Kommunikation mit einem Bediener umfasst. Es wird vorgeschlagen, dass das zumindest eine Bedienerendgerät zu einer Kommunikation mit der Blendschutzvorrichtung zumindest eine Kommunikationseinheit und zumindest eine mit der Kommunikationseinheit gekoppelte Lichtquelle aufweist. Vorzugsweise ist die Kommunikationseinheit des Bedienerendgeräts Teil einer Recheneinheit des Bedienerendgeräts. Ferner sind verschiedene, einem Fachmann als sinnvoll erscheinende Lichtquellen denkbar, wie beispielsweise ein Blitzlicht des Bedienerendgeräts, ein Display des Bedienerendgeräts und/oder eine externe Lichtquelle. Unter einer "Eingabeeinheit" soll hier insbesondere eine Einheit verstanden werden, die zumindest ein Bauteil aufweist, das direkt von einem Bediener betätigbar ist, und die in zumindest einem Betriebszustand dazu vorgesehen ist, durch eine Betätigung und/oder durch eine Eingabe von Parametern einen Prozess und/oder einen Zustand einer mit der Eingabeeinheit gekoppelten Einheit zu beeinflussen und/oder zu ändern. Vorzugsweise weist die Eingabeeinheit hierfür insbesondere zumindest ein Bedienelement auf. Dabei soll unter einem "Bedienelement" insbesondere ein Element verstanden werden, das dazu vorgesehen ist, bei einem Bedienvorgang eine Eingabegröße von einem Bediener aufzunehmen und insbesondere unmittelbar von einem Bediener kontaktiert zu werden, wobei ein Berühren des Bedienelements und/oder eine auf das Bedienelement ausgeübte Betätigungskraft sensiert und/oder mechanisch zur Betätigung einer Einheit weitergeleitet wird. Es sind verschiedene, einem Fachmann als sinnvoll erscheinende Bedienelemente denkbar, insbesondere soll darunter jedoch ein Touchscreen verstanden werden. Unter einer "Ausgabeeinheit" soll in diesem Zusammenhang insbesondere eine Einheit zu einer Ausgabe von Informationen an einen Bediener verstanden werden. Vorzugsweise soll darunter insbesondere eine Einheit zu einer Darstellung von Informationen für einen Erfinder verstanden werden. Es sind verschiedene, einem Fachmann als sinnvoll erscheinende Ausgabeeinheiten denkbar, wie beispielsweise eine Statusleuchte und/oder ein Display. Dadurch kann insbesondere eine vorteilhaft zuverlässige Kommunikation bereitgestellt werden. Durch die Kommunikation zwischen der Blendschutzvorrichtung und dem Bedienerendgerät können über das Bedienerendgerät beispielsweise Informationen abgerufen oder Einstellungen vorgenommen werden. Heutige Blendschutzvorrichtungen verfügen über eine große Anzahl an verschiedenen Funktionen und Einstellmöglichkeiten. Diese sind bei den meisten Blendschutzvorrichtung über Bedienelemente außen oder innen an der Blendschutzvorrichtung angebracht. Meist ist der Platz begrenzt und die Funktionsvielfalt gross, sodass die Beschriftung und Gestaltung der Bedienoberfläche schwierig und meist zu klein ausfällt. Es wurden bereits viele Versuche unternommen die Bedienung intuitiv und einfach zu gestalten. Dazu wurde auch bei einigen Helmen komplett auf gewisse Funktionen verzichtet. Bei vielen Blendschutzvorrichtungen werden Einstellungen jedoch sehr wenig bis gar nicht vorgenommen. Meist werden nach dem Kauf die Funktionen einmalig oder gar nicht eingestellt und dann für den Rest des Lebenszyklus so belassen. Durch die Kommunikation könnte insbesondere eine bedienelementlose Blendschutzvorrichtung bereitgestellt werden. Die Möglichkeit mit dem Smartphone aber trotzdem diverse Funktionen einzustellen eröffnet neue Perspektiven in der Verkaufsargumentation.

Des Weiteren wird vorgeschlagen, dass die zumindest eine Kommunikationseinheit des Bedienerendgeräts zu einer Kommunikation mit der Blendschutzvorrichtung dazu vorgesehen ist, die Lichtquelle zu einer Erzeugung von Lichtimpulsen anzusteuern. Vorzugsweise weisen die mittels der Lichtquelle erzeugten Lichtimpulse eine Impulsrate von zumindest 5 Hz, vorzugsweise von zumindest 20 Hz und besonders bevorzugt von zumindest 30 Hz auf. Dadurch kann insbesondere eine vorteilhaft zuverlässige und schnelle Datenübertragung ermöglicht werden. Dadurch kann insbesondere eine Datenübertragung ermöglicht werden, welche sinnvoll von dem zumindest einen Sensor der Blendschutzsensoreinheit erfasst werden kann, insbesondere auch, da der Sensor für eine Arbeitszustandserfassungsfunktion bereits eine schnelle Reaktionszeit benötigt. Die Lichtquelle kann dabei teilweise auch von dem Bedienerendgerät getrennt ausgebildet sein. Beispielsweise wäre denkbar, dass die Lichtquelle von einer Beleuchtung, wie beispielsweise einer Deckenbeleuchtung, gebildet ist. Dabei wäre denkbar, dass beispielsweise auch während eines Betriebs Informationen über die Deckenbeleuchtung auf die Blendschutzvorrichtung übertragen werden. Hierdurch können beispielsweise aktuelle Informationen beispielsweise in einem Head-up Display der Blendschutzvorrichtung dargestellt werden. Ferner wäre zudem denkbar, dass beispielsweise über den Lichtbogen des Schweissgeräts Informationen an die Schweissmaske gesendet werden können.

Es wird ferner vorgeschlagen, dass das zumindest eine Bedienerendgerät zumindest einen mit der Kommunikationseinheit gekoppelten Bildsensor aufweist, welcher dazu vorgesehen ist, mittels des Blendschutzfilters übertragene Daten der Blendschutzvorrichtung zu erfassen. Vorzugsweise ist der Bildsensor dazu vorgesehen, eine Abdunkelung des optischen Blendschutzfilters der Blendschutzvorrichtung zu erfassen. Besonders bevorzugt ist der Bildsensor dazu vorgesehen, Abdunklungsimpulse des optischen Blendschutzfilters der Blendschutzvorrichtung zu erfassen. Es sind verschiedene, einem Fachmann als sinnvoll erscheinende Bildsensoren denkbar, wie beispielsweise eine bestehende Kamera des Bedienerendgeräts, ein bestehender Helligkeitssensor des Bedienerendgeräts und/oder ein extern angeschlossener Bildsensor. Dadurch kann insbesondere eine vorteilhafte Datenübertragung ermöglicht werden. Hierdurch kann insbesondere eine vorteilhafte Datenübertragung bereitgestellt werden, bei welcher Daten mittels eines Bildsensors des zumindest einen Bedienerendgeräts erfasst werden können.

Es wird weiter vorgeschlagen, dass das zumindest eine Bedienerendgerät von einem mobilen Endgerät, insbesondere einem Smartphone, gebildet ist. Dadurch kann insbesondere ein vorteilhaftes Bedienerendgerät bereitgestellt werden. Hierdurch kann insbesondere ein Bedienerendgerät bereitgestellt werden, welches in der Regel nicht extra erworben werden muss und bereits über die notwenigen Bauteile verfügt. Besonders bevorzugt kann dadurch insbesondere ein Bedienerendgerät bereitgestellt werden, welches vorteilhaft erweiter- und/oder anpassbar ist, wie beispielsweise mittels Apps und/oder externen Geräten, welche an die bestehenden Anschlüsse des Smartphones angeschlossen werden können.

Ferner geht die Erfindung aus von einem Verfahren zu einem Betrieb des System. Es wird vorgeschlagen, dass das zumindest eine Bedienerendgerät in einem ersten Verfahrensschritt Daten kodiert durch mittels einer Lichtquelle des Bedienerendgeräts erzeugte Lichtimpulse auf die Blendschutzvorrichtung überträgt. Vorzugsweise werden die Daten in Form von Lichtimpulsen, welche die Daten in Form einer Folge von Einsen und Nullen darstellen, übertragen. Dadurch kann insbesondere eine vorteilhaft zuverlässige und schnelle Datenübertragung ermöglicht werden.

Des Weiteren wird vorgeschlagen, dass die Blendschutzvorrichtung die Lichtimpulse des Bedienerendgeräts mittels einer Blendschutzsensoreinheit, welche zu einer Erfassung eines Arbeitszustands vorgesehen ist, erfasst. Vorzugsweise erfasst die Blendschutzvorrichtung die Lichtimpulse des Bedienerendgeräts mittels des zumindest einen Sensors der Blendschutzsensoreinheit, welcher zu einer Erfassung eines Arbeitszustands vorgesehen ist. Dadurch kann insbesondere eine Datenübertragung ermöglicht werden, welche sinnvoll von der Blendschutzsensoreinheit erfasst werden kann, insbesondere auch, da der Sensor für eine Arbeitszustandserfassungsfunktion bereits eine schnelle Reaktionszeit benötigt.

Vorzugsweise wird vorgeschlagen, dass die zumindest eine Blendschutzvorrichtung in einem zweiten Verfahrensschritt Daten kodiert durch eine Veränderung einer Durchlässigkeit eines Blendschutzfilters auf das Bedienerendgerät überträgt. Vorzugsweise überträgt die Kommunikationseinheit der Blendschutzvorrichtung die Daten kodiert in Form von Abdunklungsimpulsen, wobei mittels der Abdunklungsimpulse beispielsweise eine Folge von Einsen und Nullen dargestellt werden kann. Dadurch kann insbesondere eine Datenübertragung mittels des für einen regulären Betrieb benötigten Blendschutzfilters ermöglicht werden. Hierdurch kann insbesondere eine Anzahl von Bauteilen gering gehalten werden. Es kann insbesondere ein vorteilhaft kompakter Aufbau der Blendschutzvorrichtung erreicht werden.

Es wird ferner vorgeschlagen, dass das zumindest eine Bedienerendgerät in dem zweiten Verfahrensschritt zu einer Erfassung einer Durchlässigkeit des Blendschutzfilters mittels einer Lichtquelle den Blendschutzfilter anstrahlt. Vorzugsweise streut der Blendschutzfilter in Abhängigkeit von einer Durchlässigkeit das Licht unterschiedlich zurück bzw. reflektiert das Licht anders, was zu einem anderen Backscattering führt. Dies kann insbesondere durch den Bildsensor des Bedienerendgeräts erfasst werden. Während der Datenübertragung ist die Lichtquelle insbesondere dauernd eingeschaltet. Vorzugsweise wird das von der Lichtquelle zurückgeworfene Licht durch den Blendschutzfilter mit den Daten moduliert. Dadurch kann insbesondere eine vorteilhaft zuverlässige Datenübertragung erreicht werden. Es kann insbesondere eine Datenübertragung ermöglicht werden, ohne dass die Blendschutzvorrichtung hierfür einen separaten Aktor benötigt.

Die erfindungsgemäße Blendschutzvorrichtung, das System sowie das Verfahren sollen hierbei nicht auf die oben beschriebene Anwendung und Ausführungsform beschränkt sein. Insbesondere können die erfindungsgemäße Blendschutzvorrichtung, das System sowie das Verfahren zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen sind sechs Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein System mit einer Blendschutzvorrichtung, welche einen optischen Blendschutzfilter, eine Blendschutzsensoreinheit und eine Kommunikationseinheit umfasst, und mit einem Bedienerendgerät, welches eine Kommunikationseinheit umfasst, in einem Empfangszustand in einer schematischen Darstellung,
- Fig. 2: das System mit der Blendschutzvorrichtung und mit dem Bedienerendgerät in einem Übertragungszustand in einer schematischen Darstellung,
- Fig. 3: den optischen Blendschutzfilter und die Blendschutzsensoreinheit der Blendschutzvorrichtung in einer schematischen Darstellung,
- Fig. 4: ein schematisches Ablaufdiagramm eines Verfahrens zu einem Betrieb des Systems,
- Fig. 5: ein alternatives System mit einer Blendschutzvorrichtung und mit einem Bedienerendgerät in einem Empfangszustand in einer schematischen Darstellung,
- Fig. 6: das alternative System mit der Blendschutzvorrichtung und mit dem Bedienerendgerät in einem Übertragungszustand in einer schematischen Darstellung,
- Fig. 7: ein Bedienerendgerät eines weiteren alternativen Systems mit einem externen Gerät bei einem Sendevorgang in einer schematischen Darstellung,
- Fig. 8: ein Bedienerendgerät eines weiteren alternativen Systems mit einem externen Gerät bei einem Sendevorgang in einer schematischen Darstellung,
- Fig. 9: ein Bedienerendgerät eines weiteren alternativen Systems mit einem externen Gerät bei einem Empfangsvorgang in einer schematischen Darstellung und
- Fig. 10: ein Bedienerendgerät eines weiteren alternativen Systems mit einem externen Gerät bei einem Empfangsvorgang in einer schematischen Darstellung.

### Beschreibung der Ausführungsbeispiele

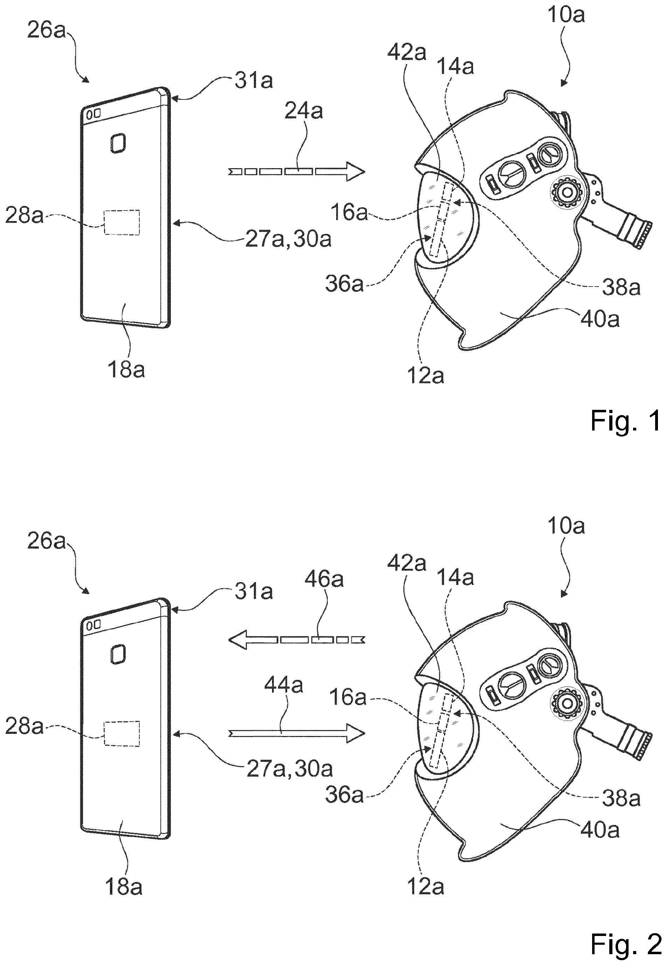

Die Figuren 1 und 2 zeigen eine Blendschutzvorrichtung 10a und ein Bedienerendgerät 18a. Die Blendschutzvorrichtung 10a und das Bedienerendgerät 18a bilden ein System 26a aus. Die Blendschutzvorrichtung 10a ist dazu vorgesehen, während eines Betriebs von einem Bediener auf dem Kopf getragen zu werden. Die Blendschutzvorrichtung 10a ist von einem Schweißhelm gebildet. Grundsätzlich wäre jedoch auch eine andere, einem Fachmann als sinnvoll erscheinende Ausbildung der Blendschutzvorrichtung 10a denkbar. Das Bedienerendgerät 18a ist von der Blendschutzvorrichtung 10a getrennt ausgebildet. Das Bedienerendgerät 18a ist von einem mobilen Endgerät gebildet. Das Bedienerendgerät 18a ist von einem Smartphone gebildet. Grundsätzlich wäre jedoch auch eine andere, einem Fachmann als sinnvoll erscheinende Ausbildung des Bedienerendgeräts 18a denkbar, wie beispielsweise als Tablet-Computer. Das Bedienerendgerät 18a weist eine Ein- und Ausgabeeinheit 27a zu einer Kommunikation mit dem Bediener auf. Die Ein- und Ausgabeeinheit 27a des Bedienerendgeräts 18a ist von einem Touchscreen gebildet. Grundsätzlich wäre jedoch eine andere, einem Fachmann als sinnvoll erscheinende Ausbildung der Ein- und Ausgabeeinheit 27a denkbar.

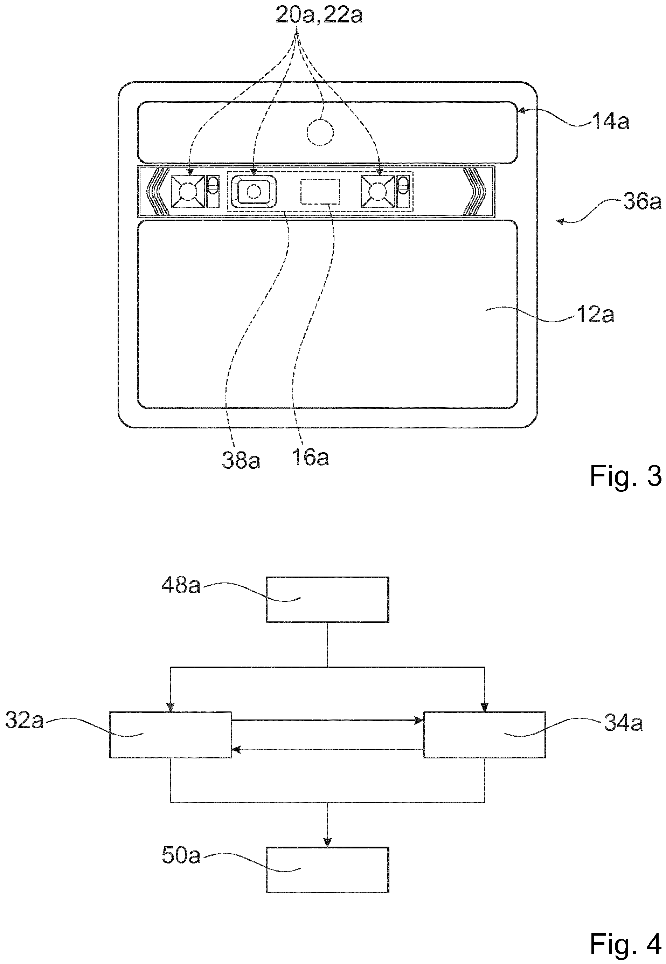

Die Blendschutzvorrichtung 10a weist einen optischen Blendschutzfilter 12a auf. Ferner weist die Blendschutzvorrichtung 10a eine Blendschutzsensoreinheit 14a auf. Die Blendschutzsensoreinheit 14a ist zu einer Erfassung eines Arbeitszustands und einer davon abhängigen Ansteuerung einer Durchlässigkeit des Blendschutzfilters 12a vorgesehen. Die Blendschutzsensoreinheit 14a weist zumindest einen Sensor 20a auf. Die Blendschutzsensoreinheit 14a weist einen Sensor 20a auf. Der Sensor 20a ist in einem regulären Betrieb dazu vorgesehen einen Schweißvorgang oder das Auftreten eines hellen Lichts, welches die Augen eines Benutzers schädigen oder auf andere Weise beeinflussen könnte, zu detektieren. Der Sensor 20a der Blendschutzsensoreinheit 14a ist von einer Photodiode oder einer Solarzelle gebildet. Grundsätzlich wäre jedoch auch eine andere, einem Fachmann als sinnvoll erscheinende Ausbildung des Sensors 20a denkbar. Des Weiteren weist die Blendschutzvorrichtung 10a eine Schutzkassette 36a auf. Der optische Blendschutzfilter 12a und die Blendschutzsensoreinheit 14a bilden einen Teil der Schutzkassette 36a. Ferner weist die Schutzkassette 36a eine Recheneinheit 38a auf. Die Recheneinheit 38a ist mit der Blendschutzsensoreinheit 14a gekoppelt. Die Recheneinheit 38a ist dazu vorgesehen, die Daten der Blendschutzsensoreinheit 14a zu verarbeiten und abhängig davon den Blendschutzfilter 12a anzusteuern. Die Recheneinheit 38a umfasst zu einer Energieversorgung eine nicht weiter sichtbare Batterie und/oder Solarzelle (Figur 3).

Der optische Blendschutzfilter 12a ist von einem elektrooptischen Filter gebildet. Der optische Blendschutzfilter 12a ist von einem automatic darkening filter, kurz ADF, gebildet. Der optische Blendschutzfilter 12a besteht aus mehreren Schichten. Der optische Blendschutzfilter 12a ist als ein Mehrschichtverbund ausgebildet. Die Flüssigkristallschicht des Blendschutzfilters 12a wird während eines regulären Betriebs von der Recheneinheit 38a abgedunkelt, wenn über die Blendschutzsensoreinheit 14a ein Schweißverfahren oder ein Lichtblitz erfasst wird. Der Blendschutzfilter 12 weist eine rechteckige Grundform auf.

Ferner weist die Blendschutzvorrichtung 10a eine Schildeinheit 40a auf. Der Blendschutzfilter 12a ist fest in der Schildeinheit 40a aufgenommen. Der Blendschutzfilter 12a ist positionsfest in der Schildeinheit 40a aufgenommen. Die Schutzkassette 36a ist positionsfest in der Schildeinheit 40a aufgenommen. Die Blendschutzvorrichtung 10a weist ferner eine Vorsatzscheibe 42a auf. Die Vorsatzscheibe 42a ist mit der Schildeinheit 40a über nicht weiter sichtbare Rastelemente verbunden. Die Vorsatzscheibe 42a ist transparent ausgebildet. Die Vorsatzscheibe 42a ist zu einem Schutz der Schutzkassette 36a vorgesehen. Die Vorsatzscheibe 42a verdeckt die Schutzkassette 36a von außen.

Des Weiteren weist die Blendschutzvorrichtung 10a eine Kommunikationseinheit 16a auf. Die Kommunikationseinheit 16a ist zu einer Kommunikation mit dem von der Blendschutzvorrichtung 10a getrennten Bedienerendgerät 18a vorgesehen. Die Kommunikationseinheit 16a ist in die Recheneinheit 38a integriert. Die Kommunikationseinheit 16a bildet ein Teil der Recheneinheit 38a. Grundsätzlich wäre jedoch auch eine separate Ausbildung der Kommunikationseinheit 16a denkbar. Über die Kommunikationseinheit 16a können an dem Bedienerendgerät 18a Einstellungen an der Blendschutzvorrichtung 10a vorgenommen werden oder beispielsweise Betriebsstatistiken der Blendschutzvorrichtung 10a abgerufen werden. Beispielsweise können die maximale Nutzungstemperatur, die Schweißstunden, ein nächstes Serviceintervall, Batterieladung, Serienummer, Kaufdatum oder alle ADF Einstellungen ausgelesen werden. Auch ein Anpassen dieser Parameter teilweise sogar außerhalb der normalen Bedienungsmöglichkeiten wird möglich. Dies kann z.B. für den Parameter Delay, also die Zeitverzögerung nach dem Schweißen bis zum Öffnen des Blendschutzfilters 12a, genutzt werden, um unterschiedliche, insbesondere auch extreme, Verzögerungen einzustellen. Dies kann für Spezialprozesse oder für persönliche Vorlieben des Kunden genutzt werden. Ferner kann dadurch auf eine Vielzahl von Bedienelementen an der Blendschutzvorrichtung 10a verzichtet werden. Ferner lassen sich für OEM-Kunden spezifische Inhalte auf der Blendschutzvorrichtung 10a speichern, die dann von den Kunden mittels des Bedienerendgeräts 18a abgerufen werden könnten. Hierbei lassen sich beispielsweise Informationen über eine mitgelieferte Schweißmaschine, Links zur Konfigurationshilfe im Design des OEM-Kunden, Zertifikate, Links zu Ersatzteilen und Zubehör oder spezielle Gutschriften für Onlineshops abrufen.

Die Kommunikationseinheit 16a weist zu einem Empfang von Daten einen Sensor 22a auf. Der Sensor 20a der Blendschutzsensoreinheit 14a ist zumindest teilweise einstückig mit einem Sensor 22a der Kommunikationseinheit 16a ausgebildet. Der Sensor 20a der Blendschutzsensoreinheit 14a bildet den Sensor 22a der Kommunikationseinheit 16a aus. Der Sensor 20a der Blendschutzsensoreinheit 14a ist bifunktional ausgebildet. Der Sensor 20a der Blendschutzsensoreinheit 14a weist eine Arbeitszustandserfassungsfunktion und eine Kommunikationsfunktion auf. Die Kommunikationseinheit 16a ist in einem Betriebszustand dazu vorgesehen, eine Sensorkenngröße des Sensors 20a der Blendschutzsensoreinheit 14a zu einer Kommunikation zu nutzen. Die Sensorkenngröße des Sensors 20a der Blendschutzsensoreinheit 14a wird sowohl zu einer Erfassung eines Arbeitszustands, als auch zu einer Kommunikation genutzt.

Der Sensor 20a der Blendschutzsensoreinheit 14a ist in zumindest einem Empfangszustand dazu vorgesehen, mittels Licht übertragene Daten des Bedienerendgeräts 18a zu empfangen. Der Sensor 20a der Blendschutzsensoreinheit 14a ist in zumindest einem Empfangszustand der Kommunikationsfunktion dazu vorgesehen, mittels Licht übertragene Daten des Bedienerendgeräts 18a zu empfangen. Der Sensor 20a der Blendschutzsensoreinheit 14a ist in zumindest einem Empfangszustand dazu vorgesehen, kodierte Daten, welche von dem Bedienerendgerät 18a mittels Licht ausgesendet werden, zu empfangen. Der Sensor 20a der Blendschutzsensoreinheit 14a ist dazu vorgesehen ist, mittels des Bedienerendgeräts 18a erzeugte Lichtimpulse 24a zu erfassen und zu einer Auswertung an die Kommunikationseinheit 16a zu übertragen. Die Lichtimpulse 24a werden dabei von dem Sensor 20a der Blendschutzsensoreinheit 14a erfasst und ein Messsignal des Sensors 20a an die Kommunikationseinheit 16a übertragen. Die Kommunikationseinheit 16a ist in einem Betriebszustand dazu vorgesehen, die Messsignale des Sensors 20a auszuwerten und eine hinterlegte Information auszulesen und zu verarbeiten. Die in Form von Lichtimpulsen 24a übertragenen Daten werden beispielsweise als eine Folge von Einsen und Nullen kodiert, welche wiederum als Lichtimpulse 24a dargestellt werden.

Des Weiteren weist das Bedienerendgerät 18a zu einer Kommunikation mit der Blendschutzvorrichtung 10a eine Kommunikationseinheit 28a auf. Die Kommunikationseinheit 28a des Bedienerendgeräts 18a ist zu einem Austausch von Daten und/oder Informationen mit der Blendschutzvorrichtung 10a vorgesehen. Die Kommunikationseinheit 28a des Bedienerendgeräts 18a ist zu einem Austausch von Daten und Informationen mit der Kommunikationseinheit 16a der Blendschutzvorrichtung 10a vorgesehen. Ferner weist das Bedienerendgerät 18a eine mit der Kommunikationseinheit 28a gekoppelte Lichtquelle 30a auf. Die Lichtquelle 30a ist von einem Display des Bedienerendgeräts 18a gebildet. Grundsätzlich wäre jedoch auch eine andere, einem Fachmann als sinnvoll erscheinende Ausbildung der Lichtquelle 30a denkbar. Die Kommunikationseinheit 28a des Bedienerendgeräts 18a ist zu einer Kommunikation mit der Blendschutzvorrichtung 10a dazu vorgesehen, die Lichtquelle 30a zu einer Erzeugung von Lichtimpulsen 24a anzusteuern. Die Kommunikationseinheit 28a des Bedienerendgeräts 18a ist zu einer Übertragung von Daten auf die Blendschutzvorrichtung 10a dazu vorgesehen, die Lichtquelle 30a zu einer Erzeugung von Lichtimpulsen 24a anzusteuern.

Mit der Lichtquelle 30a des Bedienerendgeräts 18a kann der Übertragungsweg zu dem Blendschutzfilter 12a der Blendschutzvorrichtung 10a aufgebaut werden. Die Daten werden in einem ON-OFF-Verfahren übertragen. Die Lichtquelle 30a wird entsprechend der zu übertragenden Daten binär angesteuert. Die Lichtquelle 30a wird daher entsprechend der vorliegenden Daten ein- und ausgeschaltet. Weiterhin besteht die Möglichkeit, der Lichtquelle 30a verschiedene Intensitätswerte zuzuweisen. Mit der Modulation der Displayintensität ist eine höhere Datenrate realisierbar. Es können mehrere Zustände einem entsprechenden Intensitätswert zugewiesen werden. Bei der Datenübertragung kann während des Kommunikationsvorganges das Bedienerendgerät 18a nicht bedient werden, da kein Sichtkontakt zum Display besteht. Es sind verschiedene, einem Fachmann als sinnvoll erscheinende Möglichkeiten zum Initiieren der Verbindung denkbar. Es wäre beispielsweise denkbar, durch eine Mehrfachbetätigung eines Einschalters der Blendschutzvorrichtung 10a in einen Empfangsmodus umzuschalten (Figur 2).

Zur Erhöhung der Stabilität der Verbindung sind redundante Sendeverfahren oder Datenüberprüfungsverfahren denkbar.

Ferner ist die Kommunikationseinheit 16a in einem Übertragungszustand zu einer Übertragung von Daten auf das Bedienerendgerät 18a zu einer Ansteuerung einer Durchlässigkeit des Blendschutzfilters 12a vorgesehen. Die Kommunikationseinheit 16a ist in dem Übertragungszustand dazu vorgesehen, zu übertragende Daten kodiert durch eine Veränderung einer Durchlässigkeit des Blendschutzfilters 12a darzustellen. Die Daten werden dabei in Form von Abdunklungsimpulsen kodiert. Die in Form von Abdunklungsimpulsen übertragenen Daten werden beispielsweise als eine Folge von Einsen und Nullen kodiert, welche wiederum als Abdunklungsimpulse dargestellt werden.

Des Weiteren weist das Bedienerendgerät 18a einen mit der Kommunikationseinheit 28a gekoppelten Bildsensor 31 a auf. Der Bildsensor 31 a ist dazu vorgesehen, mittels des Blendschutzfilters 12a übertragene Daten der Blendschutzvorrichtung 10a zu erfassen. Der Bildsensor 31 a ist dazu vorgesehen, bei einer Übertragung von Daten von der Blendschutzvorrichtung 10a auf das Bedienerendgerät 18a, die mittels des Blendschutzfilters 12a übertragenen Daten der Blendschutzvorrichtung 10a zu erfassen. Der Bildsensor 31 a ist von einem Umgebungslichtsensor des Bedienerendgeräts 18a gebildet. Grundsätzlich wäre jedoch auch denkbar, dass der Bildsensor 31 a beispielsweise von einer Frontkamera des Bedienerendgeräts 18a gebildet ist.

Zu einer Kommunikation der Blendschutzvorrichtung 10a mit dem Bedienerendgerät 18a wird der Blendschutzfilter 12a gezielt geöffnet und geschlossen. Somit kann der Blendschutzfilter 12a entsprechend der zu übertragenden Daten angesteuert werden. Der Blendschutzfilter 12a kann mit der Lichtquelle 30a des Bedienerendgeräts 18a angestrahlt werden. Der Blendschutzfilter 12a wird mit der Lichtquelle 30a des Bedienerendgeräts 18a mit einem konstanten Lichtstrahl 44a angestrahlt. In Abhängigkeit vom Zustand des Blendschutzfilters 12a wird das Licht unterschiedlich zurückgestreut bzw. reflektiert. Die Reflektionen 46a können mittels dem Bildsensor 31 a erfasst werden. Während der Datenübertragung ist die Lichtquelle 30a dauernd eingeschaltet. Das von der Lichtquelle 30a zurückgeworfene Licht wird durch den Blendschutzfilter 12a mit den Daten moduliert. Mit dem Bildsensor 31 a kann eine Änderung der Lichtintensität der Umgebung festgestellt werden. Damit ist es möglich, Daten von dem Blendschutzfilter 12a in Richtung des Bedienerendgeräts 18a zu übertragen. Durch die Verwendung des Displays des Bedienerendgeräts 18a als Lichtquelle 30a kann mit unterschiedlichen Farben beleuchtet werden. Damit kann der Einfluss des Umgebungslichts minimiert werden und die optischen Eigenschaften des Blendschutzfilters 12a können besser ausgenutzt werden (Figur 3).

Figur 4 zeigt ein schematisches Ablaufdiagramm eines Verfahrens zu einem Betrieb des Systems 26a. Das Verfahren beschreibt vereinfacht eine mögliche Kommunikation zwischen dem Bedienerendgerät 18a und der Blendschutzvorrichtung 10a. Bei dem Verfahren findet sowohl eine Übertragung von Daten von dem Bedienerendgerät 18a auf die Blendschutzvorrichtung 10a statt, als auch eine Übertragung von Daten von der Blendschutzvorrichtung 10a auf das Bedienerendgerät 18a. Das Verfahren ist daher lediglich beispielhaft. Grundsätzlich wäre auch denkbar, dass bei einer Kommunikation lediglich eine Übertragung von Daten von dem Bedienerendgerät 18a auf die Blendschutzvorrichtung 10a oder eine Übertragung von Daten von der Blendschutzvorrichtung 10a auf das Bedienerendgerät 18a stattfindet.

Zu Beginn des Verfahrens wird in einem Verfahrensschritt 48a eine Verbindung initiiert. Dies kann beispielsweise durch eine Mehrfachbetätigung eines Einschalters der Blendschutzvorrichtung 10a erfolgen. Anschließend folgt entweder ein erster Verfahrensschritt 32a, in welchem Daten von dem Bedienerendgerät 18a auf die Blendschutzvorrichtung 10a übertragen werden, oder ein zweiter Verfahrensschritt 34a, in welchem Daten von der Blendschutzvorrichtung 10a auf das Bedienerendgerät 18a übertragen werden.

Bei dem Verfahren kodiert das Bedienerendgerät 18a in einem ersten Verfahrensschritt 32a Daten durch mittels der Lichtquelle 30a des Bedienerendgeräts 18a erzeugte Lichtimpulse 24a auf die Blendschutzvorrichtung 10a. Die Blendschutzvorrichtung 10a erfasst die Lichtimpulse 24a des Bedienerendgeräts 18a mittels der Blendschutzsensoreinheit 14a, welche in einem Betriebszustand zu einer Erfassung eines Arbeitszustands vorgesehen ist. Die Blendschutzvorrichtung 10a erfasst die Lichtimpulse 24a des Bedienerendgeräts 18a mittels des Sensors 20a der Blendschutzsensoreinheit 14a. Hierdurch können Daten des Bedienerendgeräts 18a, wie beispielsweise an dem Bedienerendgerät 18a vorgenommene Einstellungen für die Blendschutzvorrichtung 10a, auf die Blendschutzvorrichtung 10a übertragen werden.

Ferner überträgt die Blendschutzvorrichtung 10a in einem zweiten Verfahrensschritt 34a Daten kodiert durch eine Veränderung einer Durchlässigkeit eines Blendschutzfilters 12a auf das Bedienerendgerät 18a. Das Bedienerendgerät 18a strahlt dazu in dem zweiten Verfahrensschritt 34a zu einer Erfassung einer Durchlässigkeit des Blendschutzfilters 12a mittels einer Lichtquelle 30a den Blendschutzfilter 12a an. Die Reflektionen 46a der Lichtquelle 30a werden anschließend von dem Bildsensor 31 a des Bedienerendgeräts 18a erfasst. Hierdurch können Daten der Blendschutzvorrichtung 10a, wie beispielsweise aktuelle Betriebszeiten, auf das Bedienerendgerät 18a übertragen werden.

Nach einer Übertragung der Daten in dem ersten Verfahrensschritt 32a und/oder dem zweiten Verfahrensschritt 34a wird die Verbindung zwischen der Blendschutzvorrichtung 10a und dem Bedienerendgerät 18a in einem Verfahrensschritt 50a getrennt.

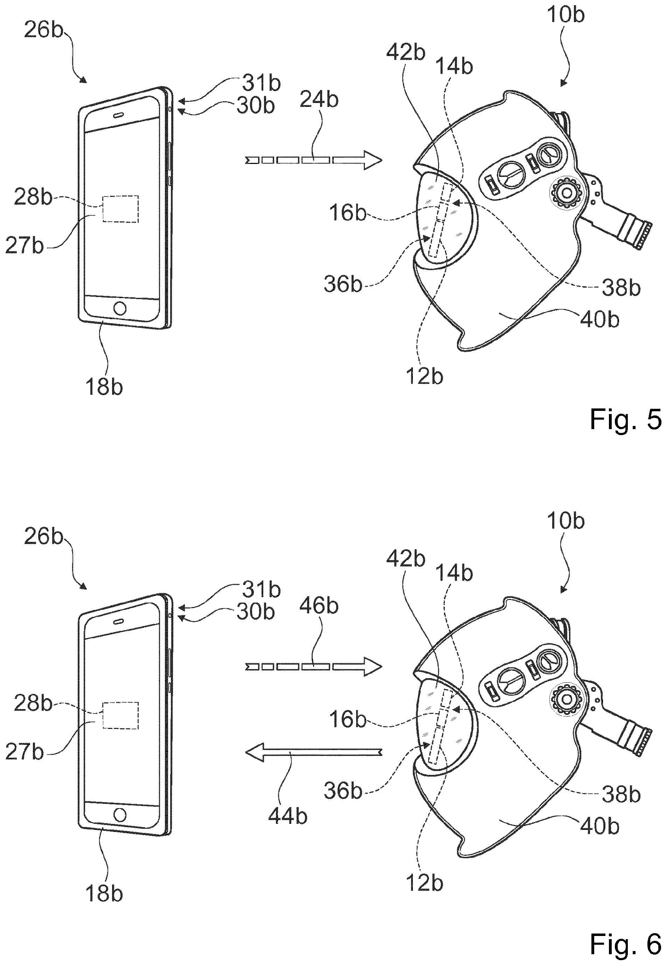

In den Figuren 5 bis 10 sind fünf weitere Ausführungsbeispiele der Erfindung gezeigt. Die nachfolgenden Beschreibungen beschränken sich im Wesentlichen auf die Unterschiede zwischen den Ausführungsbeispielen, wobei bezüglich gleichbleibender Bauteile, Merkmale und Funktionen auf die Beschreibung der anderen Ausführungsbeispiele, insbesondere der Figuren 1 bis 4, verwiesen werden kann. Zur Unterscheidung der Ausführungsbeispiele ist der Buchstabe a in den Bezugszeichen des Ausführungsbeispiels der Figuren 1 bis 4 durch die Buchstaben b bis f in den Bezugszeichen der Ausführungsbeispiele der Figuren 5 bis 10 ersetzt. Bezüglich gleich bezeichneter Bauteile, insbesondere in Bezug auf Bauteile mit gleichen Bezugszeichen, kann grundsätzlich auch auf die Zeichnungen und/oder die Beschreibung der anderen Ausführungsbeispiele, insbesondere der Figuren 1 bis 4, verwiesen werden.

Figur 5 zeigt eine Blendschutzvorrichtung 10b und ein Bedienerendgerät 18b. Die Blendschutzvorrichtung 10b und das Bedienerendgerät 18b bilden ein System 26b aus. Die Blendschutzvorrichtung 10b ist von einem Schweißhelm gebildet. Das Bedienerendgerät 18b ist von einem mobilen Endgerät gebildet. Das Bedienerendgerät 18b ist von einem Smartphone gebildet.

Das Bedienerendgerät 18b weist zu einer Kommunikation mit der Blendschutzvorrichtung 10b eine Kommunikationseinheit 28b auf. Die Kommunikationseinheit 28b des Bedienerendgeräts 18b ist zu einem Austausch von Daten und/oder Informationen mit der Blendschutzvorrichtung 10b vorgesehen. Ferner weist das Bedienerendgerät 18b eine mit der Kommunikationseinheit 28b gekoppelte Lichtquelle 30b auf. Die Lichtquelle 30b ist von einem Blitzlicht, insbesondere von einem Kamerablitzlicht, des Bedienerendgeräts 18b gebildet. Die Kommunikationseinheit 28b des Bedienerendgeräts 18b ist zu einer Kommunikation mit der Blendschutzvorrichtung 10b dazu vorgesehen, die Lichtquelle 30b zu einer Erzeugung von Lichtimpulsen 24b anzusteuern. Mit der Lichtquelle 30b des Bedienerendgeräts 18b kann der Übertragungsweg zu dem Blendschutzfilter 12b der Blendschutzvorrichtung 10b aufgebaut werden. Die Daten werden in einem ON-OFF-Verfahren übertragen. Die Lichtquelle 30b wird entsprechend der zu übertragenden Daten binär angesteuert. Die Lichtquelle 30b wird daher entsprechend der vorliegenden Daten ein- und ausgeschaltet (Figur 5).

Des Weiteren weist das Bedienerendgerät 18b einen mit der Kommunikationseinheit 28b gekoppelten Bildsensor 31 b auf. Der Bildsensor 31 b ist dazu vorgesehen, mittels des Blendschutzfilters 12b übertragene Daten der Blendschutzvorrichtung 10b zu erfassen. Der Bildsensor 31 b ist dazu vorgesehen, bei einer Übertragung von Daten von der Blendschutzvorrichtung 10b auf das Bedienerendgerät 18b die mittels des Blendschutzfilters 12b übertragenen Daten der Blendschutzvorrichtung 10b zu erfassen. Der Bildsensor 31 b ist von einer Kamera des Bedienerendgeräts 18b gebildet. Der Bildsensor 31 b ist von einer Kamera auf einer Rückseite des Bedienerendgeräts 18b gebildet. Zu einer Kommunikation der Blendschutzvorrichtung 10b mit dem Bedienerendgerät 18b wird ein Blendschutzfilter 12b der Blendschutzvorrichtung 10a gezielt geöffnet und geschlossen. Somit kann der Blendschutzfilter 12b entsprechend der zu übertragenden Daten angesteuert werden. Der Blendschutzfilter 12b kann mit der Lichtquelle 30b des Bedienerendgeräts 18b angestrahlt werden. Der Blendschutzfilter 12b wird mit der Lichtquelle 30b des Bedienerendgeräts 18b mit einem konstanten Lichtstrahl 44b angestrahlt. In Abhängigkeit vom Zustand des Blendschutzfilters 12b wird das Licht unterschiedlich zurückgestreut bzw. reflektiert. Die Reflektionen 46b können mittels des Bildsensors 31 b erfasst werden. Während der Datenübertragung ist die Lichtquelle 30b dauernd eingeschaltet. Das von der Lichtquelle 30b zurückgeworfene Licht wird durch den Blendschutzfilter 12b mit den Daten moduliert. Mit dem Bildsensor 31 b des Bedienerendgeräts 18a kann eine Änderung der Lichtintensität der Umgebung festgestellt werden. Damit ist es möglich, Daten von dem Blendschutzfilter 12b in Richtung des Bedienerendgeräts 18b zu übertragen (Figur 6).

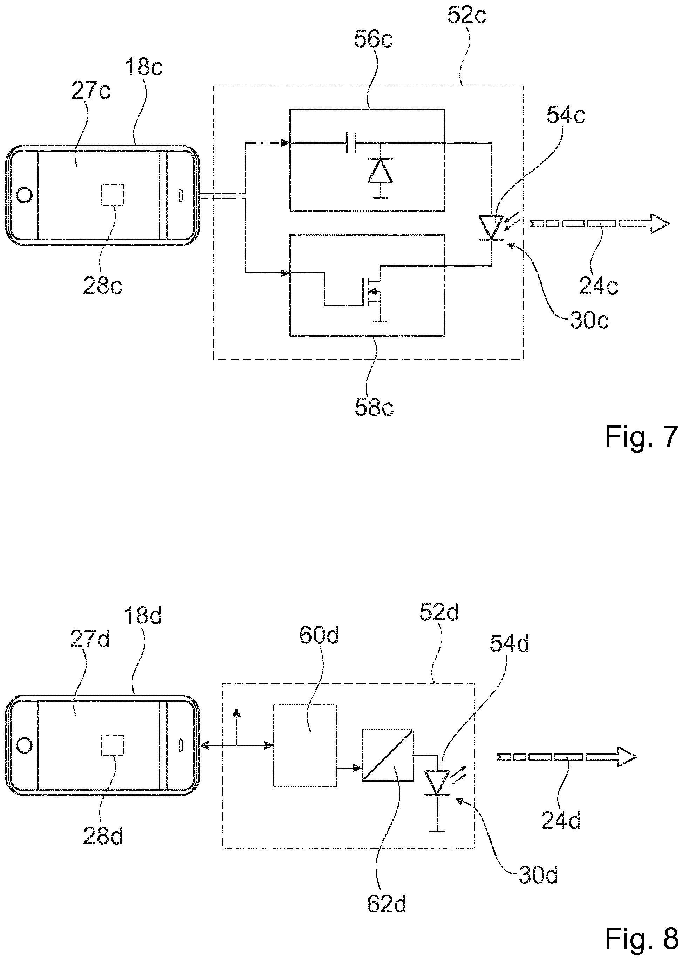

Figur 7 zeigt ein Bedienerendgerät 18c eines Systems. Das Bedienerendgerät 18c weist ein externes Gerät 52c auf. Das externe Gerät 52c weist eine LED 54c auf. Das externe Gerät 52c ist an einem nicht weiter sichtbaren Kopfhöreranschluss des Bedienerendgeräts 18c angeschlossen. Über das externe Gerät 52c können Daten von dem Bedienerendgerät 18c auf eine Blendschutzvorrichtung übertragen werden. Der Kopfhöreranschluss des Bedienerendgeräts 18c kann neben der Audioübertragung auch zur Datenübertragung genutzt werden. Die zu übertragenden digitalen Daten können in ein analoges Audiofile konvertiert werden. Es kann z.B. ein FSK-moduliertes Signal erzeugt werden. Die binären Zustände werden zwei analogen Sinusträgern zugeordnet. Dieses modulierte Signal kann am Kopfhörerausgang des Bedienerendgeräts 18c ausgegeben werden. Die Ausgangsleistungen eines Kopfhörerverstärkers des Bedienerendgeräts 18c liegen in einem Bereich von wenigen mW. Mit dem Kopfhörerverstärker kann somit energetisch die Ansteuerung der LED 54c ermöglicht werden. Die LED 54c benötigt eine Flussspannungen von zumindest 1,8 V. Die Ausgangsspannung an dem Kopfhörerausgang des Bedienerendgeräts 18c liegt bei maximaler Lautstärke in einem Bereich von 300 mVᵣₘₛ bzw. 420 mVₚₑₐₖ bei Ausgangsströmen von ca. 150 mArms. Eine direkte Ansteuerung der LED 54c ist somit nicht möglich, da die Ausgangsspannung zu gering ist. Das externe Gerät 52c weist hierfür eine kapazitive Ladungspumpe 56c, insbesondere eine Greinacher-Schaltung, auf. Durch die Kaskadierung mehrerer Kondensatoren und Dioden lässt sich aus einer Wechselspannung eine Gleichspannung erzeugen, die ein Vielfaches der Eingangsspitzenspannung beträgt. Dazu wird an einem Audiokanal ein konstantes Sinussignal mit maximaler Amplitude ausgegeben und als Quellenspannung für die Ladungspumpe 56c benutzt. Grundsätzlich wäre jedoch auch denkbar, durch eine phasenverdrehte Ansteuerung des linken und rechten Kanals zwischen den beiden Kanälen eine doppelte Spitzenspannung zu erzeugen. Ferner besteht grundsätzlich die Möglichkeit, mit einem Audiokanal einen DC/DC Konverter bzw. eine Spannungsvervielfachung zu realisieren. Es ist auch der Einsatz eines Transformators mit nachfolgender Gleichrichtung möglich. Damit kann eine höhere Spannung zur Verfügung gestellt werden. Die kapazitive Ladungspumpe 56c ist an einen ersten Audiokanal des Kopfhörerausgangs des Bedienerendgeräts 18c angeschlossen. An dem ersten Audiokanal liegt eine konstante Spannung mit einer konstanten Frequenz an. Ferner weist das externe Gerät 52c einen Switch 58c auf, welcher an einen zweiten Audiokanal des Kopfhörerausgangs des Bedienerendgeräts 18c angeschlossen ist. Über den zweiten Audiokanal werden die Daten in einem ON-OFF-Verfahren übertragen. Entsprechend der zu übertragenden Daten wird die LED 54c amplitudenmoduliert bzw. ein- und ausgeschalten. Es ist auch die Umsetzung einer Frequenzmodulation denkbar.

Figur 8 zeigt ein Bedienerendgerät 18d eines Systems. Das Bedienerendgerät 18d weist ein externes Gerät 52d auf. Das Gerät 52d weist eine LED 54d auf. Das Gerät 52d ist an einem nicht weiter sichtbaren USB-Anschluss des Bedienerendgeräts 18d angeschlossen. Der USB-Anschluss ist von einem USB on-the-go Anschluss gebildet. Über das externe Gerät 52d können Daten von dem Bedienerendgerät 18c auf eine Blendschutzvorrichtung übertragen werden. Über den USB-Anschluss kann das externe Gerät 52d mit einem Hauptgerät des Bedienerendgeräts 18d kommunizieren. Durch das Programmieren des Bedienerendgeräts 18d, beispielsweise mittels einer Applikation, kann das Bedienerendgerät 18d in einem USB Host Mode das externe Gerät 52d ansteuern. In Verbindung mit einem geeigneten USB-UART-Wandler 60d kann damit eine serielle Schnittstelle bereitgestellt werden. Das externe Gerät 52d weist den USB-UART-Wandler 60d auf. Nach dem USB-UART-Wandler 60d ist zudem ein V/I-Wandler 62d des externen Geräts 52d angeordnet. Zusätzlich kann das externe Gerät 52d mit der Spannung von dem USB-Anschluss versorgt werden. Über eine Applikation des Bedienerendgeräts 18d können über eine virtuelle serielle Schnittstelle die zu übertragenden Daten ausgegeben werden. Diese Daten werden an einem Ausgang des USB-UART-Wandlers 60d als serielle Daten ausgegeben. Mit einer spannungsgesteuerten Stromquelle kann dieser Ausgang die LED 54d betreiben. Entsprechend den vorliegenden Daten wird diese dann ein- und ausgeschaltet.

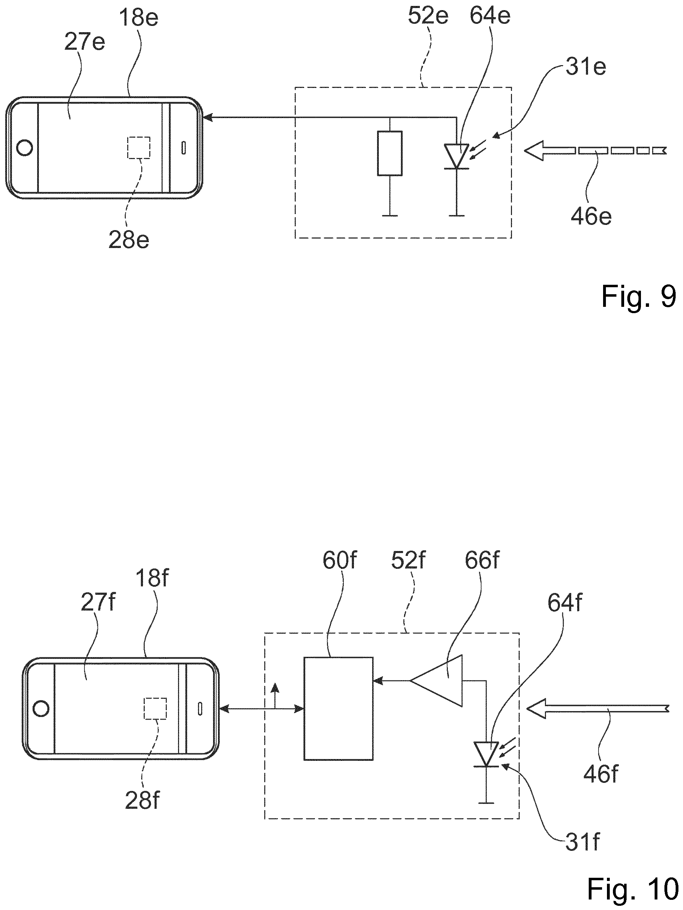

Figur 9 zeigt ein Bedienerendgerät 18e eines Systems. Das Bedienerendgerät 18e weist ein externes Gerät 52e auf. Das Gerät 52e weist eine Photodiode 64e auf. Grundsätzlich wäre jedoch auch denkbar, dass das Gerät 52e eine Solarzelle aufweist. Das Gerät 52e ist an einem nicht weiter sichtbaren Kopfhöreranschluss des Bedienerendgeräts 18e angeschlossen. Über das externe Gerät 52e können Daten von einer Blendschutzvorrichtung auf das Bedienerendgerät 18e übertragen werden. Der Kopfhöreranschluss kann neben der Kommunikationsrichtung vom Bedienerendgerät 18e zu einer Blendschutzvorrichtung auch zum Empfang von Daten genutzt werden. Neben einem Kopfhörerausgang weist der Kopfhöreranschluss ein Mikrofoneingang auf. Somit kann ein kompaktes Transceiver-Modul konzipiert werden. Bei dem Mikrofoneingang handelt es sich um einen hochohmigen analogen Eingang, welcher zur Erfassung von Spannungsänderungen eingesetzt werden kann. Bei Verwendung der Photodiode 64e im Solarzellenbetrieb wird keine zusätzliche Spannung benötigt. Die Photodiode 64e liefert eine zur einfallenden Lichtstärke proportionale Ausgangsspannung. Die Spannungsänderungen lassen sich am Mikrofoneingang erfassen und entsprechend auswerten. Die Photodiode 64e ist dazu vorgesehen, bei einer Übertragung von Daten von der Blendschutzvorrichtung auf das Bedienerendgerät 18e, die mittels eines Blendschutzfilters übertragenen Daten der Blendschutzvorrichtung zu erfassen. Der Blendschutzfilter kann mit einer nicht weiter sichtbaren LED des externen Geräts 52e des Bedienerendgeräts 18e angestrahlt werden. Der Blendschutzfilter wird mit der LED des Bedienerendgeräts 18e mit einem konstanten Lichtstrahl angestrahlt. In Abhängigkeit vom Zustand des Blendschutzfilter wird das Licht unterschiedlich zurückgestreut bzw. reflektiert. Die Reflektionen 46e können mittels der Photodiode 64e erfasst werden. Während der Datenübertragung ist die LED dauernd eingeschaltet. Die LED wird mit einer maximalen Helligkeit betrieben. Grundsätzlich wäre auch die Verwendung des Blitzlichts wie auch des Displays des Bedienerendgeräts 18e als Lichtquelle denkbar.

Figur 9 zeigt ein Bedienerendgerät 18f eines Systems. Das Bedienerendgerät 18f weist ein externes Gerät 52f auf. Das Gerät 52f weist eine Photodiode 64f auf. Das Gerät 52f ist an einem nicht weiter sichtbaren USB-Anschluss des Bedienerendgeräts 18f angeschlossen. Über das externe Gerät 52f können Daten von einer Blendschutzvorrichtung auf das Bedienerendgerät 18f übertragen werden. An dem USB-Anschluss kann neben dem Sender auch ein Empfänger umgesetzt werden. Das Schaltungsprinzip des externen Geräts 52f ist entsprechend dem des externen Geräts 52d der Figur 8 ausgeführt, wobei der gleiche USB-UART-Wandler 60f verwendet wird. Nunmehr wird der Empfangspfad des USB-UART-Wandler 60f benutzt. Mit der Photodiode 64f wird das von dem Blendschutzfilter der Blendschutzvorrichtung rückgestreute Licht empfangen. Das Signal der Photodiode 64f wird verstärkt, damit es von dem USB-UART-Wandler 60f verwertet werden kann. Das externe Gerät 52f weist dazu einen Verstärker 66f auf. Die Photodiode 64f ist dazu vorgesehen, bei einer Übertragung von Daten von der Blendschutzvorrichtung auf das Bedienerendgerät 18f, die mittels eines Blendschutzfilters übertragenen Daten der Blendschutzvorrichtung zu erfassen. Der Blendschutzfilter kann mit einer nicht weiter sichtbaren LED des externen Geräts 52f des Bedienerendgeräts 18f angestrahlt werden. Der Blendschutzfilter wird mit der LED des Bedienerendgeräts 18f mit einem konstanten Lichtstrahl angestrahlt. In Abhängigkeit vom Zustand des Blendschutzfilters wird das Licht unterschiedlich zurückgestreut bzw. reflektiert. Die Reflektionen 46f können mittels der Photodiode 64f erfasst werden. Während der Datenübertragung ist die LED dauernd eingeschaltet. Die LED wird mit einer maximalen Helligkeit betrieben. Grundsätzlich wäre auch die Verwendung des Blitzlichts wie auch des Displays des Bedienerendgeräts 18f als Lichtquelle denkbar.

## Patentansprüche

1. Blendschutzvorrichtung, insbesondere Schweißhelm, mit zumindest einem optischen Blendschutzfilter (12a; 12b), mit zumindest einer Blendschutzsensoreinheit (14a; 14b), welche zu einer Erfassung eines Arbeitszustands und einer davon abhängigen Ansteuerung einer Durchlässigkeit des Blendschutzfilters (12a; 12b) vorgesehen ist, und mit zumindest einer Kommunikationseinheit (16a; 16b) zu einer Kommunikation mit einem von der einen Blendschutzvorrichtung (10a; 10b) getrennten Bedienerendgerät (18a; 18b; 18c; 18d; 18e; 18f),
**dadurch gekennzeichnet, dass**
die zumindest eine Blendschutzsensoreinheit (14a; 14b) zumindest einen Sensor (20a) aufweist, der zumindest teilweise einstückig mit einem Sensor (22a) der Kommunikationseinheit (16a; 16b) ausgebildet ist.

2. Blendschutzvorrichtung nach Anspruch 1,
**dadurch gekennzeichnet, dass**
die zumindest eine Kommunikationseinheit (16a; 16b) in zumindest einem Betriebszustand dazu vorgesehen ist, eine Sensorkenngröße des zumindest einen Sensors (20a) der zumindest einen Blendschutzsensoreinheit (14a; 14b) zu einer Kommunikation zu nutzen.

3. Blendschutzvorrichtung nach Anspruch 1 oder 2,
**dadurch gekennzeichnet, dass**
der zumindest eine Sensor (20a) der zumindest einen Blendschutzsensoreinheit (14a; 14b) bifunktional ausgebildet ist und zumindest eine Arbeitszustandserfassungsfunktion und zumindest eine Kommunikationsfunktion aufweist.

4. Blendschutzvorrichtung nach einem der vorhergehenden Ansprüche,
**dadurch gekennzeichnet, dass**
der zumindest eine Sensor (20a) der zumindest einen Blendschutzsensoreinheit (14a; 14b) in zumindest einem Empfangszustand dazu vorgesehen ist, mittels Licht übertragene Daten des Bedienerendgeräts (18a; 18b; 18c; 18d; 18e; 18f) zu empfangen.

5. Blendschutzvorrichtung nach Anspruch 4,
**dadurch gekennzeichnet, dass**
der zumindest eine Sensor (20a) der zumindest einen Blendschutzsensoreinheit (14a; 14b) dazu vorgesehen ist, mittels des Bedienerendgeräts (18a; 18b; 18c; 18d; 18e; 18f) erzeugte Lichtimpulse (24a; 24b; 24c; 24d) zu erfassen und zu einer Auswertung an die zumindest eine Kommunikationseinheit (16a; 16b) zu übertragen.

6. Blendschutzvorrichtung nach einem der vorhergehenden Ansprüche,
**dadurch gekennzeichnet, dass**
der zumindest eine Sensor (20a) der zumindest einen Blendschutzsensoreinheit (14a; 14b) von einer Photodiode oder einer Solarzelle gebildet ist.

7. Blendschutzvorrichtung nach einem der vorhergehenden Ansprüche,
**dadurch gekennzeichnet, dass**
die zumindest eine Kommunikationseinheit (16a; 16b) in zumindest einem Übertragungszustand zu einer Übertragung von Daten auf das Bedienerendgerät (18a; 18b; 18c; 18d; 18e; 18f) zu einer Ansteuerung einer Durchlässigkeit des Blendschutzfilters (12a; 12b; 12c) vorgesehen ist.

8. System mit zumindest einer Blendschutzvorrichtung (10a; 10b) nach einem der vorhergehenden Ansprüche, mit zumindest einem von der zumindest einen Blendschutzvorrichtung (10a; 10b) getrennten Bedienerendgerät (18a; 18b; 18c; 18d; 18e; 18f), welches zumindest eine Ein- und/oder Ausgabeeinheit (27a; 27b; 27c; 27d; 27e; 27f) zu einer Kommunikation mit einem Bediener umfasst.

9. System nach Anspruch 8,
**dadurch gekennzeichnet, dass**
das zumindest eine Bedienerendgerät (18a; 18b; 18c; 18d; 18e; 18f) zu einer Kommunikation mit der Blendschutzvorrichtung (10a; 10b) zumindest eine Kommunikationseinheit (28a; 28b; 28c; 28d; 28e; 28f) und zumindest eine mit der Kommunikationseinheit (28a; 28b; 28c; 28d; 28e; 28f) gekoppelte Lichtquelle (30a; 30b; 30c; 30d) aufweist.

10. System nach Anspruch 9,
**dadurch gekennzeichnet, dass**
die zumindest eine Kommunikationseinheit (28a; 28b; 28c; 28d; 28e; 28f) des Bedienerendgeräts (18a; 18b; 18c; 18d; 18e; 18f) zu einer Kommunikation mit der Blendschutzvorrichtung (10a; 10b) vorgesehen ist, die Lichtquelle (30a; 30b; 30c; 30d) zu einer Erzeugung von Lichtimpulsen (24a; 24b; 24c; 24d) anzusteuern.

11. System nach Anspruch 9 oder 10,
**dadurch gekennzeichnet, dass**
das zumindest eine Bedienerendgerät (18a; 18b; 18c; 18d; 18e; 18f) zumindest einen mit der Kommunikationseinheit (28a; 28b; 28c; 28d; 28e; 28f) gekoppelten Bildsensor (31 a; 31 b; 31 e; 31 f) aufweist, welcher dazu vorgesehen ist, mittels des Blendschutzfilters (12a; 12b) übertragene Daten der Blendschutzvorrichtung (10a; 10b) zu erfassen.

12. System nach einem der Ansprüche 8 bis 11,
**dadurch gekennzeichnet, dass**
das zumindest eine Bedienerendgerät (18a; 18b; 18c; 18d; 18e; 18f) von einem mobilen Endgerät, insbesondere einem Smartphone, gebildet ist.

13. Verfahren zu einem Betrieb des Systems (26a; 26b) nach einem der Ansprüche 8 bis 12.

14. Verfahren nach Anspruch 13,
**dadurch gekennzeichnet, dass**
das zumindest eine Bedienerendgerät (18a; 18b; 18c; 18d; 18e; 18f) in einem ersten Verfahrensschritt (32a) Daten kodiert durch mittels einer Lichtquelle (30a; 30b; 30c; 30d) des Bedienerendgeräts (18a; 18b; 18c; 18d; 18e; 18f) erzeugte Lichtimpulse (24a; 24b; 24c; 24d) auf die Blendschutzvorrichtung (10a; 10b) überträgt.

15. Verfahren nach Anspruch 14,
**dadurch gekennzeichnet, dass**
die Blendschutzvorrichtung (10a; 10b) die Lichtimpulse (24a; 24b; 24c; 24d) des Bedienerendgeräts (18a; 18b; 18c; 18d; 18e; 18f) mittels einer Blendschutzsensoreinheit (14a; 14b), welche zu einer Erfassung eines Arbeitszustands vorgesehen ist, erfasst.

16. Verfahren nach einem der Ansprüche 13 bis 15,
**dadurch gekennzeichnet, dass**
die zumindest eine Blendschutzvorrichtung (10a; 10b) in einem zweiten Verfahrensschritt (34a) Daten kodiert durch eine Veränderung einer Durchlässigkeit eines Blendschutzfilters (12a; 12b) auf das Bedienerendgerät (18a; 18b; 18c; 18d; 18e; 18f) überträgt.

17. Verfahren nach Anspruch 16,
**dadurch gekennzeichnet, dass**
das zumindest eine Bedienerendgerät (18a; 18b; 18c; 18d; 18e; 18f) in dem zweiten Verfahrensschritt (34a) zu einer Erfassung einer Durchlässigkeit des Blendschutzfilters (12a; 12b) mittels einer Lichtquelle (30a; 30b; 30c; 30d) den Blendschutzfilter (12a; 12b) anstrahlt.
